# DEMANDE DE BREVET EUROPEEN

(11) **EP 1 096 245 A1**
(43) Date de publication de la demande: **02.05.2001**
(21) Numéro de dépôt: 00402972.4
(22) Date de dépôt: 26.10.2000
(51) Int. Cl.: G01N 1/22, A61B 5/083

(54) **Procédé et dispositif de receuil d'air expiré aux fins d'analyses**

(30) Priorité: 29.10.1999 FR 9913582
(71) Demandeur: AR21 SA - Analyses, Recherches et Innovation Instrumentale, 92140 Clamart (FR)
(72) Inventeur: Delvorde, Pascal, 91300 Massy (FR); Massias, Laurent, 75015 Paris (FR); Sarbach, Christian, 78000 Versailles (FR)
(74) Mandataire: Doireau, Marc

(57) **Abrégé**

L'invention vise à permettre des mesures fiables portant essentiellement sur l'air alvéolaire en disposant d'un matériel transportable. Pour ce faire, l'invention propose de recueillir les dernières fractions d'air expiré sur plusieurs expirations successives en concentrant ces fractions par adsorption par un pompage d'air déclenché à la fin de chaque expiration.

Selon un exemple de mise en oeuvre, des moyens (1 à 3) d'inhalation d'air ambiant et d'expulsion d'un flux d'air inhalé conduisent l'air vers une pièce de séparation (5) à deux voies (5a, 5b), une première voie (5a) étant raccordée à une pompe (8) de prélèvement d'air à travers un capteur (6) de commande d'un commutateur (7), et une seconde voie (5b) étant connectée à un tube (9) contenant l'air expiré à chaque expiration. Un raccord (10) est réalisé dans cette voie (5b) à proximité de la pièce de séparation (5) pour réexpirer la fraction de queue de l'air expiré dans une cartouche d'adsorption (11) disposée entre ce raccord (10) et la pompe (8), la pompe étant déclenchée et coupée par le capteur de commande (6) respectivement à la fin et au début de chaque expiration en fonction de la détection de déplacement du flux d'air expiré selon la direction initiale (F3, F4).

## Description

L'invention se rapporte à un procédé et à un dispositif de recueil d'air expiré par une personne afin d'analyser certains composés de cet air.

L'invention concerne les techniques non invasives de mesure de la dégradation des membranes cellulaires du corps humain dont certains composés forment des marqueurs évacués par l'air pulmonaire. La formation des radicaux libres et la péroxydation lipidique sont les principales causes des dommages cellulaires. En particulier, la péroxydation des acides gras polyinsaturés induit la formation de gaz éthane et de méthane, éliminés par l'air pulmonaire. Ces hydrocarbures volatiles sont considérés habituellement comme des marqueurs satisfaisants de la dégradation lipidique. Les techniques non-invasives utilisent alors l'adsorption d'un échantillon gazeux provenant de l'air pulmonaire pour mesurer par désorption la quantité d'éthane et de pentane.

Il existe de nombreuses méthodes pour le recueil de l'air alvéolaire. Les méthodes les plus courantes utilisent des tubes de type Haldane-Priestly associés à des cartouches de matériau adsorbant. D'autres techniques utilisent des sacs de stockage, par exemple des sacs en téflon®, peu pratiques pour le transport des prélèvements.

Ces méthodes présentent une grande dispersion dans les mesures obtenues. Cette dispersion provient du fait que l'expiration à travers la cartouche est difficile et n'est pas constituée que d'air alvéolaire. De plus la quantification d'hydrocarbures ne repose que sur une seule expiration à laquelle la personne s'est en général préparée.

L'invention vise à palier les inconvénients ci-dessus mentionnés, en particulier elle vise à permettre des mesures fiables portant essentiellement sur l'air alvéolaire en disposant d'un matériel transportable et d'un encombrement minimum pour être opérationnel sur tout site.

Pour atteindre ces objectifs, l'invention propose de recueillir les dernières fractions d'air expiré sur plusieurs expirations successives afin d'augmenter la quantité de volume analysé et d'éviter la dilution du prélèvement par la première fraction d'air d'origine atmosphérique présent dans la trachée.

Plus précisément, l'invention a pour objet un procédé de recueil d'air par inhalation d'air ambiant et expiration à travers un guidage tubulaire selon un flux d'expiration, caractérisé en ce qu'il consiste à prélever des fractions terminales d'expirations successives en récupérant et concentrant ces fractions par adsorption d'air, la récupération et la concentration étant réalisées en déclenchant à la fin de chaque expiration un pompage d'air en aval de l'adsorption, selon une direction inversée au flux d'expiration.

L'invention concerne également un dispositif de prélèvement pour la mise en oeuvre de ce procédé. Un tel dispositif de prélèvement d'air alvéolaire comporte des moyens d'inhalation d'air ambiant et d'expulsion d'un flux d'air inhalé qui est conduit selon une direction de déplacement vers une pièce de séparation à deux voies, une première voie étant raccordée à une pompe de prélèvement d'air à travers un capteur de commande d'un commutateur d'interruption/déclenchement de la pompe, et une seconde voie étant connectée à un tube de longueur suffisante pour contenir tout l'air expiré à chaque expiration, un raccord étant réalisé dans cette voie, à proximité de la pièce de séparation, pour réexpirer selon la direction opposée à la direction de déplacement initiale une fraction de queue de l'air expiré dans une cartouche d'adsorption disposée entre ce raccord et la pompe de prélèvement, la pompe étant déclenchée et coupée par le capteur de commande respectivement à la fin et au début de chaque expiration en fonction de la détection du déplacement du flux d'air expiré selon la direction initiale.

Selon des modes de réalisation particuliers :
- les moyens d'inhalation et d'expulsion d'air comportent une valve anti-retour de flux d'air ascendant provenant de l'air ambiant, un embout buccal d'inhalation et une valve anti-retour de flux d'air pulmonaire descendant, l'air expulsé étant conduit jusqu'à la pièce de séparation par une tubulure en matériau chimiquement inerte ;
- le capteur de commande du commutateur de la pompe est un capteur de pression, la pompe étant coupée ou déclenchée lorsque la pression détectée par le capteur respectivement dépasse ou passe sous au moins une valeur seuil de pression préétablie ;
- le débit de gaz à travers la cartouche est situé entre 200 ml et 800 ml pour ne pas éluer les composés adsorbés et limiter la durée globale du prélèvement à moins de 3 minutes afin de ne pas fatiguer l'utilisateur.

Avantageusement, les matériaux utilisés pour les tubulures sont souples et compatibles avec les composés à doser, par exemple en PVC .

Dans ces conditions, le dispositif présente un encombrement réduit puisqu'il permet d'éviter les volumes morts importants, tout en étant fiable et sûr.

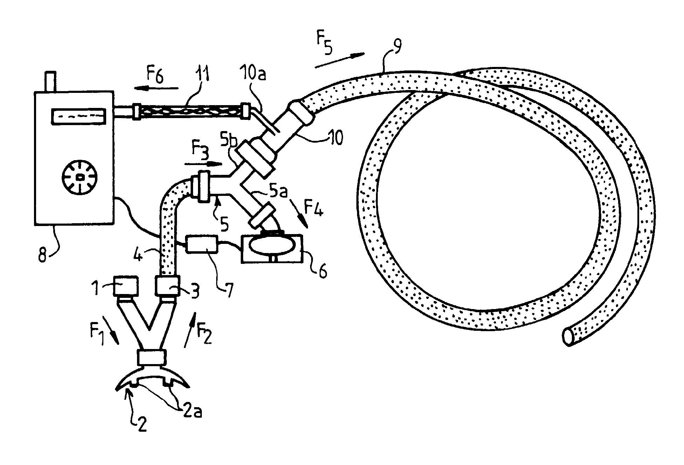

D'autres caractéristiques et avantages de l'invention ressortiront de la description ci-après d'un exemple de réalisation détaillé, illustré par la figure unique annexée qui représente un dispositif de prélèvement de l'air alvéolaire selon l'invention.

Comme il apparaît sur cette figure, l'air ambiant est inhalé à travers une valve anti-retour 1 par aspiration à travers un embout buccal 2, cet embout comportant des moyens de prise en bouche 2a. En variante, il est possible de disposer un filtre sur la valve anti-retour 1 afin d'éliminer les impuretés de l'air ambiant. Le flux d'air passant à travers la valve 1 forme le flux ascendant (flèche F1) vers l'utilisateur qui l'inhale à travers l'embout 2 puis l'expire, sous forme d'air pulmonaire, à travers une deuxième valve anti-retour 3, cet air formant un flux dit descendant (flèche F2).

Ce flux descendant est ensuite conduit par une tubulure 4 en matériau inerte, par exemple en PVC, jusqu'à une pièce de séparation 5 (flèche F3). La tubulure 4 possède un diamètre de 2,5 cm pour une longueur maximale de 15 cm.

Sur l'une des voies 5a de la pièce de séparation 5, le flux d'air (flèche F4) est dirigé vers un capteur de pression 6 qui commande un commutateur marche/arrêt 7 d'une pompe de prélèvement 8.

Sur l'autre voie 5b de la pièce de séparation 5, le flux d'air expiré (flèche F5) est dirigé vers une tubulure 9 de longueur suffisante pour accueillir l'air expiré par une même expiration. Dans l'exemple de réalisation, la tubulure 9 est de type identique à la tubulure 3, sur une longueur d'environ 25 cm.

La jonction entre la seconde voie 5b et la tubulure de recueil de l'air expiré 9, est réalisée par un raccord 10 apte à recueillir l'air expiré à travers une tuyère 10a. Cet air est pompé par la pompe de prélèvement 8 à travers une cartouche d'adsorption 11.

Le montage des différents éléments utilisés dans ce dispositif ne sort pas des compétences normales de l'homme du métier. En particulier les pièces de montage et de raccord peuvent être en matériau plastique, n'adsorbant pas ni n'émettant d'hydrocarbures, la pompe est à débit ajustable avec indicateur de débit, et la cartouche d'adsorption est formée de différentes couches de carbones graphitisés et hydrophobes.

En fonctionnement, le flux descendant d'une première expiration d'air (flèche F4) provoque l'arrêt de la pompe de prélèvement 8 par commande du capteur de pression 5 à travers le commutateur 7. A la fin de cette expiration, la surpression baisse au niveau du capteur, et lorsqu'elle descend en dessous d'un seuil réglable selon les individus, la pompe 8 est mise en action par déclenchement d'un relais contacteur intégré au capteur et qui va basculer le commutateur 7 en position marche.

Dans ces conditions, l'air expiré, qui se trouve dans la tubulure de recueil 9, est extrait de cette tubulure par la pompe de prélèvement 8 à travers la cartouche 11. Seule la fraction de queue de l'air expiré, se trouvant dans la tubulure du côté du raccord 10, est absorbée dans la cartouche 7 à travers le raccord 8. Cette fraction de queue ré-aspirée, qui est constituée d'air alvéolaire et n'est pas polluée avec l'air ambiant du début d'expiration. Avantageusement, un rapport élevé entre le diamètre et la longueur de la tubulure de recueil 9, compris par exemple entre environ 5 et 15 voire 20, environ égal à 10 dans l'exemple de réalisation, empêche le mélange d'air alvéolaire et d'air de début d'expiration.

Une nouvelle inhalation d'air ambiant et d'expiration d'air pulmonaire déclenche l'arrêt de la pompe d'aspiration 8 lorsque la pression détectée par le capteur dépasse le seuil défini précédemment, soit sensiblement avant que l'air du début de l'expiration précédente ne soit adsorbée dans la cartouche 8. En effet, la durée entre deux expirations est sensiblement inférieure à la durée de chaque expiration.

L'air alvéolaire absorbé est concentré dans la cartouche. La cartouche est ensuite désorbée par désorption thermique qui relargue pratiquement intégralement tout le soluté adsorbé. La concentration du soluté ainsi collectée est suffisante pour qu'il soit détectable. Dans l'exemple de réalisation, un volume de 400 ml est ainsi désorbé et les quantités d'éthane et de pentane éliminées par l'air pulmonaire sont mesurées par exemple par chromatographie en phase gazeuse avec détection par ionisation de flamme.

L'invention n'est pas limitée à l'exemple de réalisation décrit et représenté. Il est ainsi possible d'utiliser un masque à la place de l'embout buccal pour inhaler l'air, de commander la pompe de prélèvement par tout moyen optique de mesure de transmission lumineuse, tout moyen de mesure d'induction magnétique ou autre, capable de déclencher ou d'arrêter la pompe à la fin de chaque expiration lorsqu'un paramètre déclencheur, transmission optique ou présence de paillettes magnétiques par exemple, varie suffisamment.

## Revendications

1. Procédé de recueil d'air par inhalation d'air ambiant et expiration à travers un guidage tubulaire selon un flux d'expiration, caractérisé en ce qu'il consiste à prélever et à concentrer des fractions terminales d'expirations successives en récupérant et concentrant ces fractions par adsorption, la récupération et la concentration étant réalisées en déclenchant à la fin de chaque expiration un pompage d'air en aval de l'adsorption, selon une direction inversée au flux d'expiration.

2. Dispositif de prélèvement d'air alvéolaire pour la mise en oeuvre du procédé selon la revendication 1, comportant des moyens (1 à 3) d'inhalation d'air ambiant et d'expulsion d'un flux d'air inhalé conduit selon une direction de déplacement vers une pièce de séparation (5) à deux voies (5a, 5b), une première voie (5a) étant raccordée à une pompe (8) de prélèvement d'air à travers un capteur (6) de commande d'un commutateur (7) d'interruption/déclenchement de la pompe (8), et une seconde voie (5b) étant connectée à un tube (9) de longueur suffisante pour contenir tout l'air expiré à chaque expiration, un raccord (10) étant réalisé dans cette voie (5b) à proximité de la pièce de séparation (5) pour réexpirer selon la direction opposée (F6) à la direction de déplacement initiale (F5) une fraction de queue de l'air expiré dans une cartouche d'adsorption (11) disposée entre ce raccord (10) et la pompe (8), la pompe étant déclenchée et coupée par le capteur de commande (6) respectivement à la fin et au début de chaque expiration en fonction de la détection de déplacement du flux d'air expiré selon la direction initiale (F3, F4).

3. Dispositif de prélèvement d'air alvéolaire selon la revendication 2, dans lequel le raccord (10) entre la seconde voie (5b) et la tubulure de recueil de l'air expiré (9), comporte une tuyère (10a) à travers laquelle l'air est pompé par la pompe de prélèvement (8) pour être adsorber dans la cartouche d'adsorption (11) disposée en amont de la pompe (8).

4. Dispositif de prélèvement d'air alvéolaire selon l'une des revendications 2 ou 3, dans lequel le rapport entre le diamètre et la longueur de la tubulure de recueil (9) est compris entre environ 5 et 20 pour empêcher le mélange d'air alvéolaire et d'air de début d'expiration.

5. Dispositif de prélèvement d'air alvéolaire selon l'une quelconque des revendications 2 à 4, dans lequel les moyens d'inhalation et d'expulsion d'air comportent une valve anti-retour (1) de flux d'air ascendant provenant de l'air ambiant, un embout buccal d'inhalation (2) et une valve anti-retour (3) de flux d'air pulmonaire descendant, l'air expulsé étant conduit jusqu'à la pièce de séparation (5) par une tubulure (4) en matériau chimiquement inerte.

6. Dispositif de prélèvement d'air alvéolaire selon l'une quelconque des revendications 2 à 5, dans lequel le capteur de commande du commutateur de la pompe est un capteur de pression (6), la pompe (8) étant coupée ou déclenchée lorsque la pression détectée par le capteur respectivement dépasse ou passe sous au moins une valeur seuil de pression préétablie.

7. Dispositif de prélèvement d'air alvéolaire selon l'une quelconque des revendications 2 à 6, dans lequel le débit de gaz à travers la cartouche est situé entre 200 ml et 800 ml pour limiter la durée globale du prélèvement à moins de 3 minutes.

8. Dispositif de prélèvement d'air alvéolaire selon l'une quelconque des revendications 2 à 7, dans lequel les matériaux utilisés pour les tubulures sont souples et compatibles avec les composés à doser.

9. Dispositif de prélèvement d'air alvéolaire selon l'une quelconque des revendications 2 à 8, dans lequel est disposé un filtre sur la valve anti-retour 1 afin d'éliminer les impuretés de l'air ambiant.
